# EUROPEAN PATENT APPLICATION

(11) **EP 4 205 751 A1**
(43) Date of publication of application: **05.07.2023**
(21) Application number: 21306960.2
(22) Date of filing: 30.12.2021
(51) Int. Cl.: A61K 36/185, A61K 36/47, A61K 36/9062, A61P 27/00, A61P 27/02, A61K 36/06

(54) **PLANT EXTRACT COMPOSITION AND METHOD FOR PREPARING SUCH COMPOSITION**

(71) Applicant: Cariel, Léon, 75007 Paris (FR)
(72) Inventor: Cariel, Léon, 75007 Paris (FR)
(74) Representative: Icosa

(57) **Abstract**

The present invention relates to a herbal composition comprising: a first composition comprising an extract of *Alpinia galanga,* and a second composition comprising an extract of *Phyllanthus emblica.* The invention further relates to a process for preparing such a herbal composition. Lastly, the invention relates to the uses of the herbal composition.

## Description

### FIELD OF INVENTION

The present invention relates to a herbal composition comprising a mixture of plant extracts. The invention further relates to a process for obtaining said composition as well as to its pharmaceutical use.

### BACKGROUND OF INVENTION

Angiogenesis or vascularization is a highly regulated physiological phenomenon by which new blood vessels are produced in a tissue or organ. The so-called normal angiogenesis is activated in situations of wound healing or in the development of the fetal environment.

Pathological angiogenesis is activated in the case of cellular or metabolic disorders linked or not to age and will be responsible for potentially very disabling diseases, including, several eye diseases. The best-known pathological angiogenesis-related eye diseases comprise AMD (Age-related Macular Degeneration), retinopathy linked to diabetes, and a whole series of keratitis and glaucoma associated with an anarchic vascularization.

A number of molecules are known to have an inhibitory effect on neo-angiogenesis, such as protamine, tetrahydrocortisol, fumagellin, ascorbic acid derivatives, animal glycoproteins and cellular factors, such as interferon.

Regarding AMD treatment, the current pharmacological approaches encompass verterporfin and anti-vascular endothelial growth factors (anti-VEGF) such as ranibizumab, pegaptanib, aflibercept and brolucizumab. Verterporfin is administered intravenously, followed by light stimulation of the eye (photodynamic therapy). Anti-VEGF factors are administered by intravitreal injection.

However, the partial efficacy of these molecules, their toxicity and/or the difficulty of their administration, in particular for the intravitreal administration, hinder the patient compliance and reduce the therapeutic benefit-to-risk ratio.

Products of natural origin have also been considered in the context of AMD treatment.

Natural anti-oxidants and vitamin supplementation (combination of vitamin E, vitamin C, zinc and beta-carotene) has been marketed in France in the form of different preparations for their beneficial effect on AMD. However, the efficacy of such supplementation is limited to the early stages of AMD development. Furthermore, beta-carotene supplementation has been withdrawn due to the risk of cancer in smokers and former smokers that are vulnerable to developing AMD.

Therefore, the development of a versatile, efficient and easy to administer composition for the treatment of AMD and other diseases characterized by amyloid plaques and/or dysregulated angiogenesis remains an unmet need.

The invention aims at addressing the above shortcoming by supplying a herbal composition as hereinafter described.

### SUMMARY

The invention relates to a herbal composition comprising:
a) a first composition comprising an extract of *Alpinia galanga,* and
b) a second composition comprising an extract of *Phyllanthus emblica,* and optionally further comprising *Terminalia chebula* and *Terminalia bellirica* extracts.

According to a first embodiment, the first, and/or the second composition, are fermented compositions with at least one yeast, preferably a yeast of the Saccharomycetaceae family, even more preferably the yeast is *Saccharomyces cerevisiae.*

According to a second embodiment, the herbal composition is a fermented herbal composition with at least one yeast, preferably a yeast of the Saccharomycetaceae family, even more preferably the yeast is *Saccharomyces cerevisiae.*

In one embodiment, the herbal composition according to the invention comprises:
- from 1 g/L to 70 g/L of the first composition consisting of an extract of *Alpinia galanga,* and
- from 1 g/L to 70 g/L of the second composition comprising an extract of *Phyllanthus emblica.*

In one embodiment, the herbal composition according to the invention comprises:
- from 1 g/L to 70 g/L of the first composition consisting of an extract of *Alpinia galanga,* and
- from 10 g/L to 70 g/L of the second composition consisting of a mixture of *Phyllanthus emblica, Terminalia chebula* and *Terminalia bellirica* extracts

The invention further relates to a pharmaceutical composition comprising the herbal composition and at least one pharmaceutically acceptable excipient.

The invention also relates to a nutraceutical composition comprising the herbal composition and at least one nutraceutically acceptable excipient. The pharmaceutical composition or the nutraceutical composition according may be liquid compositions formulated in the form a vial packaging.

The invention also concerns the herbal composition or the pharmaceutical composition according the invention, for use as a drug.

In one embodiment, the drug is for use in the treatment of an ocular degenerative disease selected from the group consisting of age-related macular degeneration (AMD), severe short-sightedness, cataract, diabetic retinopathy, and glaucoma.

In one embodiment, the ocular degenerative disease is age-related macular degeneration (AMD) selected from the group consisting of early AMD, dry AMD and wet AMD, preferably AMD is wet AMD.

In one embodiment, the herbal composition or the pharmaceutical composition is orally administered to the subject at least once a day.

According to one embodiment, the herbal composition or the pharmaceutical composition is administered sequentially to or simultaneously with at least one second pharmaceutical composition being selected from i) an anti-VEGF treatment, preferably the anti-VEGF treatment being selected from ranibizumab, pegaptanib, aflibercept and brolucizumab and a combination thereof and/or ii) verterporfin.

The invention also relates to a process for preparing a herbal composition according to the invention, said process comprising the steps of:
i. supplying a first composition comprising an extract of *Alpinia galanga,*
ii. supplying a second composition comprising an extract of *Phyllanthus emblica*, and optionally further comprising *Terminalia chebula* and *Terminalia bellirica* extracts,
iii. mixing the first and the second compositions, leading to a herbal composition.

In one embodiment, the process further comprises the steps of:
iv. fermenting the herbal composition of step (iii) with at least one yeast, preferably at least one Saccharomycetaceae strain, even more preferably *Saccharomyces cerevisiae,* leading to a fermented herbal composition,
v. optionally condensing the fermented herbal composition of step (v), leading to a condensed herbal composition, and
vi. optionally mixing the fermented herbal composition of step (iv) or the condensed herbal composition of step (v) with at least one pharmaceutically and/or nutraceutically acceptable excipient.

### DEFINITIONS

In the present invention, the following terms have the following meanings:

"**About**" preceding a figure means plus or less 10*%* of the value of said figure, preferably plus or less 5*%* of the value of said figure, in particular plus or less 1 *%* of the value of said figure.

"**Excipient**" refers to any inactive ingredient which is required for the formulation of an active agent in a suitable dosage form. Excipients are materials suitable for administration and include any such material known in the art which is non-toxic and which does not interact with any components of the composition in a deleterious manner. In one embodiment, "Excipients" refers to any and all solvents, diluents, carriers, fillers, bulking agents, binders, disintegrants, polymer, lubricant, glidant, surfactants, isotonic agents, thickening or emulsifying agents, stabilizers, absorption accelerators, flavoring agents, preservatives, antioxidants, buffering agents, or any combination thereof.

"**Pharmaceutical composition**" refers to a composition comprising an active principle in association with a pharmaceutically acceptable vehicle or excipient. A pharmaceutical composition is for therapeutic use, and relates to health. Especially, a pharmaceutical composition may be indicated for treating a disease or disorder.

By "**pharmaceutically acceptable**" is meant that the ingredients of a pharmaceutical composition are compatible with each other and not deleterious to the subject to which/whom it is administered.

"**Pharmaceutically acceptable excipient**" refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA. The terms "**pharmaceutically acceptable excipient**", "**pharmaceutically acceptable carrier**" or "**pharmaceutical vehicle**" refer to an inert medium or carrier used as a solvent or diluent in which the pharmaceutically active ingredient is formulated and/or administered, and which does not produce an adverse, allergic or other reaction when administered to an animal, preferably a human being. For the purposes of the invention, "**pharmaceutically acceptable excipient**" includes all pharmaceutically acceptable excipients as well as all pharmaceutically acceptable carriers, diluents, and/or adjuvants.

The term "**therapeutically effective amount**" (or more simply "**effective amount**") refers to the amount of active agent, herein the herbal composition, alone or as part of a pharmaceutical composition, that is aimed at, without causing significant negative or adverse side effects to the subject in need of treatment, preventing, reducing, alleviating or slowing down (lessening) one or more of the symptoms of a condition, symptom, disorder or disease. "**Pharmaceutically effective amount**" refers to the amount of a pharmaceutical composition necessary and sufficient for slowing down or stopping the progression, aggravation, or deterioration of one or more symptoms of a disease, disorder or condition; or alleviating the symptoms of a disease, disorder or condition; or curing the disease, disorder or condition.

"**Nutraceutical composition**" refers to a composition comprising an edible ingredient allegedly providing a physiological benefit. A nutraceutical composition is for non-therapeutic use, and relates to comfort. The term "comfort" refers to the feeling of ease or well-being. Especially, a nutraceutical composition may be used for promoting, maintaining and/or improving comfort or for alleviating and/or preventing a discomfort. A nutraceutical composition may be in the form of a nutritional product, such as, for example, a functional food or a food or dietary supplement.

"**Nutraceutically effective amount**" refers to the amount of a nutraceutical composition, food or dietary supplement or functional food necessary and sufficient for providing a physiological benefit or alleviating a discomfort.

The term "**administration**", or a variant thereof (*e.g.*, "**administering**"), means providing the active agent, herein the herbal composition, alone or as part of a pharmaceutically and/or nutraceutically acceptable composition, to the patient in whom/which the condition, symptom, or disease is to be treated.

The term "**rhizome**" is a botanical term referring to a modified subterranean plant stem that sends out roots and shoots from its nodes.

The term "**subject**" refers to a mammal, preferably a human. According to the present invention, a subject is a mammal, preferably a human. In one embodiment, the subject is a "patient", i.e., a mammal, preferably a human, who/which is awaiting the receipt of, or is receiving medical care or was/is/will be the object of a medical procedure or is monitored for the development of a condition, symptom, disorder or disease.

The term "**human**" refers to a subject of both genders and at any stage of development (*i.e.*, neonate, infant, juvenile, adolescent, adult).

The terms "**treat**", "**treating**" or "**treatment**", as used herein, refer to a therapeutic treatment, to a prophylactic (or preventive) treatment, or to both a therapeutic treatment and a prophylactic (or preventive) treatment, wherein the object is to prevent, reduce, alleviate, and/or slow down (lessen) one or more of the symptoms of a condition, disorder or disease, in a subject in need thereof.

### DETAILED DESCRIPTION

According to a first aspect, the invention relates to a herbal composition comprising: a first composition comprising an extract of *Alpinia galanga*, and a second composition comprising an extract of *Phyllanthus emblica.*

Unless otherwise specified, the extracts herein described can be selected from whole plant, fruit, bark, leaf, flower, root and/or rhizhome extracts. Preferably, the extracts may be selected from fruit, root and/or rhizhome extracts.

The first composition comprises or consists of an extract of *Alpinia galanga.* In one embodiment, the first composition consists of an extract of *Alpinia galanga.*

*Alpinia galanga,* also known as the greater galangal, lengkuas or blue ginger, is an aromatic herbaceous edible plant belonging to the Zingiberaceae family. It is of note that *Alpinia galanga* designates a different species from *A. officinarum* that is known as lesser galangal.

*Alpinia galanga* is native from South-east Asia. The rhizome of *A. galanga* is a tuberous rhizome, reddish on the surface, that presents characteristic circular rings, with odorous rings and pungent taste.

The phytochemical analysis of *A. galanga* has shown that it is a plant rich in secondary metabolites comprising high levels of essential oil, rich in cineole. Further *A. galanga* constituents that have been identified comprise flavonoids, shogaols, neolignans and phenylpropanoids, of which the main studied is acetoxychavicol acetate (AAC). Recent articles have highlighted the anti-tumor properties of AAC in different animal models.

To the Applicant's knowledge, *A. galanga* has been reported for its potential pharmacological use against digestive and rheumatological disorders as well as viral infections.

According to a preferred embodiment, the extract is an extract of *Alpinia galanga* rhizomes.

The extract of *A. galanga,* preferably of *A. galanga* rhizomes, may be obtained by any extraction method known in the art. According to an exemplary embodiment, A. *galanga,* preferably *A. galanga* rhizomes, may be crushed, followed by a solid/liquid extraction with a solvent, providing an *A. galanga* extract. Typically, the *A. galanga* extract comprises acetoxychavicol acetate. The operation may be repeated at least twice in order to ensure the quantitative extraction of *A. galanga* metabolites. In one embodiment, the solvent is ethanol, providing an ethanolic *A. galanga* extract. In one embodiment, the solvent is methanol, providing an methanolic *A. galanga* extract. In one embodiment, the solvent is a mixture of ethanol with water, providing a hydroalcoholic *A. galanga* extract. According to one embodiment, the solvent is a mixture of ethanol with water in an ethanol:water volume ratio ranging from 40:60 to 96:4, from 50:50 to 90:10, from 60:40 to 85:15, or from 70:30 to 80:20. According to one embodiment, the solvent is 100*%* water.

The obtained extract can be concentrated by removing the solvent, such as for example by subjecting the extract to reduced pressure and optionally heat.

According to a specific embodiment, the first composition comprises or consists of an ethanolic extract of *Alpinia galanga* rhizomes.

The second composition comprises or consists of an extract of *Phyllanthus emblica.*

*Phyllanthus emblica* was previously indexed as *Emblica officinalis*, is also known as embli, emblic myrobalan, myrobalan, Indian gooseberry, Malacca tree, or amla, and is a deciduous tree of the family Phyllanthaceae. The *Phyllanthus emblica* contains high amounts of ascorbic acid and has a bitter taste that is attributed to a high density of ellagitannins, such as emblicanin A, emblicanin B, punigluconin, and pedunculagin. *Phyllanthus emblica* also contains polyphenols, such as punicafolin and phyllanemblinin A, phyllanemblin, ellagic acid, gallic acid and flavonoids, such as kaempferol. Due to its astringent taste, *Phyllanthus emblica* is commonly used in the Indian medicine.

The *Phyllanthus emblica* may be in the form of a mix know in the art as Triphala extract mix. The Triphala extract mix is used in the Indian medicine for kidney and liver dysfunctions.

Thus, in one embodiment, the second composition comprises or consists of a mixture of *Phyllanthus emblica*, *Terminalia chebula* and *Terminalia bellirica* extracts. In one embodiment, the second composition consists of a mixture of *Phyllanthus emblica*, *Terminalia chebula* and *Terminalia bellirica* extracts.

*Terminalia chebula*, commonly known as black- or chebulic myrobalan, is a deciduous tree of the Combretaceae family, native from South Asia and South-east Asia. The *Terminalia chebula* chemical composition comprises triterpenes, triterpene glycosides, phenolic compounds including ellagic acid, 2,4-chebulyl-β-*D*-glucopyranose, tannins such as chebulinic acid, gallic acid, ethyl gallate, punicalagin, terflavin A, terchebin, luteolin, and tannic acid. The dried *Terminalia chebula* fruit is also used in Indian medicine as a purported antitussive, cardiotonic, homeostatic, diuretic, and laxative.

*Terminalia bellirica*, also indexed as *Terminalia bellerica*, is also known as baheda, bahera, beleric or bastard myrobalan, and is a deciduous tree of the Combretaceae family. The fruits of *T. bellirica* are reported as a multi-use therapeutic herbal product in the Tibetan medicine and are prescribed as a general health tonic in the traditional Indian medicine. The chemical constituents of *T. bellirica* comprise tannins, ellagic acid, ethylgallate, gallylglucose, and chebulinic acid. The reported *Terminalia bellirica* uses comprise its use as antioxidant, antimicrobial, antidiarrhoeal, anticancer, antidiabetic, antihypertensive and hepatoprotective agent.

Each of the *Phyllanthus emblica, Terminalia chebula* and *Terminalia bellirica* extracts, preferably fruit extracts, may be obtained by any extraction method known in the art or as indicatively presented herein above for the first composition. In one embodiment, each of the *Phyllanthus emblica, Terminalia chebula* and *Terminalia bellirica* extracts, preferably fruit extracts, is an aqueous extract. In one embodiment, the aqueous extract is obtained with a solvent comprising at least 90*%* water, at least 95*%* water, at least 99*%* water, or 100*%* water.

Preferably, when the second composition comprises or consists of the Triphala extract mixture, the relative weight proportion of *Phyllanthus emblica, Terminalia chebula* and *Terminalia bellirica* extracts, preferably fruit extracts, typically dry fruit extracts, in the second composition is 1:1:1.

According to a specific embodiment, the second composition comprises or consists of an aqueous extract of *Phyllanthus emblica, Terminalia chebula* and *Terminalia bellirica* fruits. The second composition may be obtained by mixing *Phyllanthus emblica, Terminalia chebula* and *Terminalia bellirica*, preferably their fruits, and then extracting them as detailed above. Alternatively, *Phyllanthus emblica, Terminalia chebula* and *Terminalia bellirica,* preferably their fruits, are extracted separately as detailed above and then the obtained extracts, preferably the obtained aqueous extracts, are mixed together to form Triphala or the second composition according to the present invention.

In one embodiment, the herbal composition does not comprise an extract of Bacopa spp. plant. In one embodiment, the herbal composition does not comprise an extract of *Bacopa monnieri.*

Advantageously, the first and the second compositions are fermented compositions with at least one yeast. In one embodiment, the first and/or the second compositions are fermented compositions with at least one yeast

In one first variant, the first and the second compositions are fermented compositions with said at least one yeast.

Thus, in one typical embodiment, the herbal composition is a fermented herbal composition. It should be understood that, in the context of the present specification, the term "extract" can refer to the embodiments of the invention wherein the (first and/or second ) extract is fermented as well as to the embodiments wherein said extract is not fermented. Likewise, in the context of the present specification, the term "herbal composition" can refer to the embodiments of the invention wherein the herbal composition is fermented as well as the embodiments wherein the herbal composition is not fermented.

In one embodiment, the first and the second compositions are fermented with the same at least one yeast. In another embodiment, each of the first and the second extracts is fermented with a different yeast.

According to a preferred embodiment, the at least one yeast is a yeast of the Saccharomycetaceae family. Indicative Saccharomycetaceae family genera can be selected from the group consisting of *Brettanomyces, Candida, Debaromyces, Kluyveromyces, Pichia, Torulaspora, Zygosaccharomyces* and *Saccharomyces.*

Preferably, the at least one yeast is at least one *Saccharomyces* yeast. The at least one *Saccharomyces* genus yeast may be selected from the group consisting of *Saccharomyces cerevisiae, S. cerevisiae* var *boulardii, S. arboricolus, S. bayanus, S. coriocanus, S. chevallierun S. eubayanus, S. florentinus, S. kudriavzevii, S. mikatae, S. paradoxus, S. pastorianus,* and *S. uvarum.*

Even more preferably the at least one yeast is *Saccharomyces cerevisiae. Saccharomyces cerevisiae,* also known as brewers' yeast, is commonly used in winemaking, baking, and brewing. *S. cerevisiae* is also reported as a food complement. The oral use of *Saccharomyces cerevisiae* is generally recognized as safe.

According to a first variant, the first and the second compositions are fermented separately with the at least one yeast prior to be mixed in order to form the herbal composition according to the invention. According to a second and preferred variant, the first and the second compositions are mixed together prior to being (jointly) fermented with the at least one yeast.

The herbal composition according to the invention may be solid, liquid or in the form of a paste. In one embodiment, the herbal composition is liquid or in the form of a paste, preferably the herbal composition is liquid. It should be understood that the state of the herbal composition is described at atmospheric pressure and at room temperature (25°C).

In one embodiment, the amount of the first composition ranges from 5 *%* to 60, from 5 to 50*%*, or from 10 to 50 *%* in weight relative to the total weight of the herbal composition. In one embodiment, the amount of the first composition ranges from 10*%* to 20 *%* in weight relative to the total weight of the herbal composition.

In one embodiment, the amount of the second composition ranges from 5 *%* to 60*%*, from 5 to 50*%*, or from 10 to 50 *%* in weight relative to the total weight of the herbal composition. In one embodiment, the amount of the second composition ranges from 20 *%* to 60 *%* in weight relative to the total weight of the herbal composition.

In one embodiment, the amount of the first and/or the second composition ranges from 5 *%* to 60 *%*, from 5 to 50*%*, or from 10 to 50 *%* in weight relative to the total weight of the herbal composition. It is understood that the sum of the weight percentages does not exceed 100*%*.

In one embodiment, the herbal composition according the invention comprises or consists of:
- from 5 *%* to 60 *%*, or from 10 to 20 *%* in weight relative to the total weight of the herbal composition of the first composition comprising or consisting of *Alpinia galanga* extract, preferably *A. galanga* rhizome extract, even more preferably *A. galanga* rhizome ethanolic extract, and
- from 5 *%* to 60 *%*, or from 20 to 60 *%* in weight relative to the total weight of the herbal composition of the second composition comprising or consisting of a *Phyllanthus emblica* extract, preferably fruit extract, even more preferably *Phyllanthus emblica* fruit aqueous extract.

In one embodiment, the herbal composition according the invention comprises or consists of:
- from 5 *%* to 60 *%*, or from 10 to 20 *%* in weight relative to the total weight of the herbal composition of the first composition comprising or consisting of *Alpinia galanga* extract, preferably *A. galanga* rhizome extract, even more preferably *A. galanga* rhizome ethanolic extract, and
- from 5 *%* to 60 *%*, or from 20 to 60 *%* in weight relative to the total weight of the herbal composition of the second composition comprising or consisting of a mixture of *Phyllanthus emblica, Terminalia chebula* and *Terminalia bellirica* extracts, preferably fruit extracts, even more preferably fruit aqueous extracts.

In one embodiment, the herbal composition or the fermented herbal composition comprises from 1 g/L to 20 g/L, from 5 g/L to 15 g/L, from 1 g/L to 10 g/L, or about 10 g/L of the first composition comprising or consisting of the extract of *Alpinia galanga,* preferably *A. galanga* rhizome extract, even more preferably *A. galanga* rhizome ethanolic extract. In one embodiment, the herbal composition or the fermented herbal composition comprises from about 5 g/L to about 10 g/L, of the first composition comprising or consisting of the extract of *Alpinia galanga,* preferably *A. galanga* rhizome extract, even more preferably *A. galanga* rhizome ethanolic extract. In one embodiment, the herbal composition or the fermented herbal composition comprises about 5 g/L of the first composition comprising or consisting of the extract of *Alpinia galanga,* preferably *A. galanga* rhizome extract, even more preferably *A. galanga* rhizome ethanolic extract.

In one embodiment, the herbal composition or the fermented herbal composition comprises from 5 g/L to 70 g/L, from 10 g/L to 70 g/L, from 20 g/L to 60 g/L, from 30 g/L to 50 g/L, or about 45 g/L of the second composition comprising or consisting a *Phyllanthus emblica* extract, preferably a *Phyllanthus emblica* fruit extract, even more preferably a *Phyllanthus emblica* fruit aqueous extract. In one embodiment, the herbal composition or the fermented herbal composition comprises from about 5 g/L to about 10 g/L of the second composition comprising or consisting a *Phyllanthus emblica* extract, preferably a *Phyllanthus emblica* fruit extract, even more preferably a *Phyllanthus emblica* fruit aqueous extract. In one embodiment, the herbal composition or the fermented herbal composition comprises about 7.5 g/L of the second composition comprising or consisting a *Phyllanthus emblica* extract, preferably a *Phyllanthus emblica* fruit extract, even more preferably a *Phyllanthus emblica* fruit aqueous extract.

In one embodiment, the herbal composition or the fermented herbal composition comprises from 1 g/L to 70 g/L, from 10 g/L to 70 g/L, from 20 g/L to 60 g/L, from 30 g/L to 50 g/L, or about 45 g/L of the second composition comprising or consisting of a mixture of *Phyllanthus emblica, Terminalia chebula* and *Terminalia bellirica* extracts, preferably fruit extracts, even more preferably fruit aqueous extracts. In one embodiment, the herbal composition or the fermented herbal composition comprises from about 7 g/L to about 25 g/L of the second composition comprising or consisting of a mixture of *Phyllanthus emblica, Terminalia chebula* and *Terminalia bellirica* extracts, preferably fruit extracts, even more preferably fruit aqueous extracts. In one specific embodiment, the herbal composition or the fermented herbal composition comprises about 22.5 g/L of the second composition comprising or consisting of a mixture of *Phyllanthus emblica, Terminalia chebula* and *Terminalia bellirica* extracts, preferably fruit extracts, even more preferably fruit aqueous extracts.

In one embodiment, the herbal composition according the invention comprises or consists of:
- from 1 g/L to 70 g/L, from 1 g/L to 50 g/L or from 1 g/L to 20 g/L of the first composition of *Alpinia galanga* extract, preferably *A. galanga* rhizome extract, even more preferably *A. galanga* rhizome ethanolic extract, and
- from 1 g/L to 70 g/L of the second composition comprising a *Phyllanthus emblica* extract, preferably fruit extract, even more preferably fruit aqueous extract.

In one embodiment, the herbal composition according to the invention comprises or consists of:
- from 1 g/L to 70 g/L, from 1 g/L to 50 g/L or from 1 g/L to 20 g/L of the first composition of *Alpinia galanga* extract, preferably *A. galanga* rhizome extract, even more preferably *A. galanga* rhizome ethanolic extract, and
- from 1 g/L to 70 g/L of the second composition consisting of a mixture of *Phyllanthus emblica, Terminalia chebula* and *Terminalia bellirica* extracts, preferably fruit extracts, even more preferably fruit aqueous extracts.

In one embodiment, the herbal composition according to the invention comprises or consists of:
- from 1 g/L to 50 g/L or from 1 g/L to 20 g/L of the first composition of *Alpinia galanga* extract, preferably *A. galanga* rhizome extract, even more preferably A. *galanga* rhizome ethanolic extract,
- from 10 g/L to 70 g/L of the second composition consisting of a mixture of *Phyllanthus emblica, Terminalia chebula* and *Terminalia bellirica* extracts, preferably fruit extracts, even more preferably fruit aqueous extracts, and

In one embodiment, the herbal composition comprises or consists of:
- from 5 g/L to 10 g/L of the first composition comprising an *Alpinia galanga* extract, preferably *A. galanga* rhizome extract, even more preferably *A. galanga* rhizome ethanolic extract,
- from 7 g/L to 25 g/L of the second composition comprising an extract consisting of a mixture of *Phyllanthus emblica, Terminalia chebula* and *Terminalia bellirica* extracts, preferably fruit extracts, even more preferably fruit aqueous extracts,

In one embodiment, the herbal composition comprises or consists of:
- from 5 g/L to 10 g/L of the first composition consisting of an extract of *Alpinia galanga*, preferably *A. galanga* rhizome extract, even more preferably *A. galanga* rhizome ethanolic extract,
- from 5 g/L to 10 g/L of *Phyllanthus emblica* extract, preferably a *P. emblica* fruit extract, even more preferably a *P. emblica* fruit aqueous extract,
- from 5 g/L to 10 g/L of *Terminalia chebula* extract, preferably a *T. chebula* fruit extract, even more preferably a *T. chebula* fruit aqueous extract, and
- from 5 g/L to 10 g/L *Terminalia bellirica* fruit extract, preferably a *T. bellirica* fruit extract, even more preferably a *T. bellirica* fruit aqueous extract.

In one specific embodiment, the herbal composition comprises or consists of:
- about 5 g/L of the first composition consisting of an extract of *Alpinia galanga,* preferably *A. galanga* rhizome extract, even more preferably *A. galanga* rhizome ethanolic extract,
- about 7.5 g/L of *Phyllanthus emblica* extract, preferably a *P. emblica* fruit extract, even more preferably a *P. emblica* fruit aqueous extract,
- about 7.5 g/L of of *Terminalia chebula* extract, preferably a *T. chebula* fruit extract, even more preferably a *T. chebula* fruit aqueous extract, and
- about 7.5 g/L of *Terminalia bellirica* fruit extract, preferably a *T. bellirica* fruit extract, even more preferably a *T. bellirica* fruit aqueous extract.

The invention further relates to a pharmaceutical composition that comprises the herbal composition according to the invention. Preferably, the pharmaceutical composition comprises the herbal composition in association with at least one pharmaceutically acceptable excipient.

In one embodiment, the at least one pharmaceutically acceptable excipient is selected from protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The pharmaceutical composition may also contain conventional pharmaceutical additives and adjuvants, excipients and diluents, including, but not limited to, water, gelatin of any origin, vegetable gums, lignin sulfonate, talc, sugars, starch, gum arabic, vegetable oils, polyalkylene glycols, preservatives such as sodium benzoate and/or potassium sorbate, stabilizers, emulsifying agents, buffers, lubricants, colorants, wetting agents, fillers, and flavoring agents.

The invention further relates to a nutraceutical composition that comprises the herbal composition according to the invention. Preferably, the nutraceutical composition comprises the herbal composition in association with at least one nutraceutically acceptable excipient. Examples of nutritionally acceptable excipients include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroetheral fatty acid esters, hydroxymethyl-cellulose, polyvinylpyrrolidone, flavoring agents, preservatives such as sodium benzoate and/or potassium sorbate.

In one embodiment, the pharmaceutical or nutraceutical composition is an oral pharmaceutical or nutraceutical composition. In other terms, the pharmaceutical or the nutraceutical composition is suitable for oral administration or ingestion.

In one embodiment, the pharmaceutical or the nutraceutical composition is a liquid composition. The liquid pharmaceutical or nutraceutical composition may be formulated in the form at least one vial. The at least one vial may contain 10 mL, 15 mL, 20 mL or 30 mL of the pharmaceutical or the nutraceutical composition comprising the herbal composition as defined above.

Typically, the liquid pharmaceutical or nutraceutical composition may be formulated in the form of a packaging comprising at least one, at least five, at least ten, or at least twenty vials as described above.

It should be understood that a kit-of-parts is also in the scope of the present invention. For instance, such kit-of-parts may comprise a first part comprising the first composition, optionally in association with at least one pharmaceutically or nutraceutically acceptable excipient, and a second part comprising the second composition, optionally in association with at least one pharmaceutically or nutraceutically acceptable excipient.

The invention further relates to the non-therapeutic use of a composition or a nutraceutical composition according to the invention in the management of ocular well-being. In some embodiments, the invention relates to the use of a nutraceutical composition according to the invention for alleviating eye fatigue. In some embodiments, the invention relates to the use of a nutraceutical composition according to the invention for improving visual acuity, typically in the context of eye fatigue.

According to a second aspect, the invention relates to a process for preparing a herbal composition according to the invention.

According to a first embodiment, the process comprises the steps of:
i. supplying a first composition comprising an extract of *Alpinia galanga,*
ii. supplying a second composition comprising an extract of *Phyllanthus emblica*, and optionally further comprising *Terminalia chebula* and *Terminalia bellirica* extracts,
iii. mixing the first and the second compositions, leading to a herbal composition.

According to an embodiment, the process further comprises the steps of:
iv. fermenting the herbal composition with at least one yeast, preferably at least one Saccharomycetaceae strain, even more preferably *Saccharomyces cerevisiae,* leading to a fermented herbal composition,
v. optionally condensing the fermented herbal composition, leading to a condensed herbal composition, and
vi. optionally mixing the fermented herbal composition of step (vi) or the condensed herbal composition of step (vii) with at least one pharmaceutically and/or nutraceutically acceptable excipient.

According to one embodiment, the process comprises the steps of:
i'. supplying a first composition consisting of an extract of *Alpinia galanga,* preferably an extract of *Alpinia galanga* rhizomes,
ii'. supplying a second composition consisting of a mixture of *Phyllanthus emblica, Terminalia chebula and Terminalia bellirica* extracts, preferably a mixture of *Phyllanthus emblica, Terminalia chebula and Terminalia bellirica* fruit extracts, and
iii'. fermenting each of the first and the second compositions with at least one yeast, preferably at least one Saccharomycetaceae strain, even more preferably *Saccharomyces cerevisiae,* leading to a first fermented composition and a second fermented composition;
iv'. mixing the first and the secondfermented compositions, leading to the fermented herbal composition.

Supplying the first and the secondcompositions according to steps (i) to (iii) or steps (i') to (iii') may be carried out as described in the description of the invention's composition. Likewise, the amounts of said extracts may be as described in the description of the invention's composition.

The mixing step (iv) or (v') may be carried out by any mixing means known in the art such as for example by stirring, beating or blending the first and the secondextracts or the first and the second fermented extracts.

In one embodiment, the obtained herbal composition is a powder, a paste, a solution or a suspension. Typically, the obtained herbal composition is a paste or a suspension. Without willing to be bound by a theory, fermenting the herbal composition or the first and the second extracts leads to an enhancement of the aqueous solubility of the herbal composition due to the hydrolysis of the tannins comprised therein.

The fermenting step (iv) or (iii') may optionally comprise a first step of mixing the herbal composition or each of the first and the second compositions with an aqueous composition in order to obtain a suspension. In one embodiment, the aqueous composition is 100% water. In one embodiment, the aqueous composition comprises water, glucose and an ammonium source such as ammonium nitrate. The fermenting step (iv) or (iii') may be carried out by any means known in the art, such as for example in a bioreactor, typically under agitation, preferably at a temperature ranging from 20°C to 30°C. The fermenting step (iv) or (iii') may last at least 12 hours, at least 1 day, at least 2 days, at least 3 days, at least 4 days, at least 5 days, at least 6 days, or at least 7 days. The skilled artisan knows how to adjust the duration of the fermentation step such as for example based on the bioreactor temperature and the presence or absence of glucose and/or of ammonium sources.

In one embodiment, the obtained fermented herbal composition is a powder, a solution or a suspension. Typically, the obtained fermented herbal composition is a solution or a suspension.

The invention further relates to the herbal composition or the fermented herbal composition that is obtainable or directly obtained by any one of the above process embodiments.

In one embodiment, the herbal composition or the fermented herbal composition that is obtainable or directly obtained by any one of the above process embodiments is the herbal composition or the fermented herbal composition according to the invention as described herein above.

According to a third aspect, the invention relates to a composition according to any one of the above aspects and embodiments, for use as a drug. In one embodiment, the invention relates to the herbal composition according to any one of the above aspects and embodiments for use as a drug. In one embodiment, the invention relates to the fermented herbal composition according to any one of the above aspects and embodiments for use as a drug. In one embodiment, the invention relates to the pharmaceutical composition comprising the herbal composition or the fermented herbal composition according to any one of the above aspects and embodiments, for use as a drug. The invention further relates to the use of the composition according to the invention for the preparation of a drug.

The drug according to the invention may be for use in the prevention and/or the treatment of a degenerative disease characterized by the accumulation of pathogenic proteins such as beta-amyloid protein (Altzheimer's disease, dementia, retinal and/or macular degenerative diseases) or alpha synuclein protein (Parkinson's disease). In one embodiment, the drug is for use in the prevention and/or the treatment of Altzheimer's disease. In one embodiment, the drug is for use in the prevention and/or the treatment of dementia. In one embodiment, the drug is for use in the prevention and/or the treatment of Parkinson's disease.

The drug according to the invention may also be for use in the prevention and/or the treatment of a disease characterized by a dysregulation of vascularization, in particular of a disease characterized by increased expression of the vascular endothelial-derived growth factor (VEGF). The role of VEGF in the pathogenesis of diverse cancers and degenerative eye diseases via its pro-angiogenetic effects is well established in the art. In one embodiment, the drug is for use in the prevention and/or the treatment of a cancer characterized by neo-angiogenesis, typically a solid tumor cancer such as for example colorectal cancer, kidney cancer, primary peritoneal cancer, lung cancer such as non-small cell lung cancer, gastric or gastroesophageal adenocarcinoma, hepatic cell carcinoma, thyroid cancer, pancreatic neuroendocrine cancer, gastrointestinal stromal cancer, soft tissue sarcoma, medullary thyroid cancer.

According to one typical embodiment, the drug is for use in the prevention and/or the treatment of an ocular degenerative disease. In one embodiment, the ocular degenerative disease is characterized by the accumulation of pathogenic proteins such as beta-amyloid protein and/or excessive neovascularization, such as for example due to increased VEGF expression. In one embodiment, the drug is for use in the treatment of a degenerative eye disease characterized by a dysregulation of vascularization such as diabetic retinopathy or age-related macular degeneration as described hereinbelow.

The invention further relates to a method for treating a disease as defined above, typically an ocular degenerative disease, said method comprising the administration, preferably the oral administration, of a therapeutically effective amount of a composition according to the invention to a subject in need thereof. In one embodiment, the composition is the pharmaceutical composition according to the invention.

In one embodiment, the ocular degenerative disease is selected from the group consisting of age-related macular degeneration (AMD), severe short-sightedness, cataract, diabetic retinopathy, and glaucoma. In one embodiment, the ocular degenerative disease is selected from the group consisting of age-related macular degeneration (AMD), and severe short-sightedness. In one embodiment, the ocular degenerative disease is age-related macular degeneration (AMD).

AMD is a chronic, progressive and disabling degenerative retinal disease, which typically begins after the age of 50. It selectively affects the macula, causing a degeneration of visual retinal cells. The forms of AMD can be classified as follows:
- early onset form (around 40*%* of cases), characterized by the presence of drusens, that are tiny yellow or white accumulations/nodes of extracellular material that build up between Bruch's membrane and the retinal pigment epithelium of the eye;
- atrophic or dry form (around 40*%* of cases), characterized by changes in the epithelium pigment and thinning of the macula following the development of drusen; his evolution is slow over the years; and
- exudative or wet form (about 20*%* of cases), characterized by the development of new choroidal vessels under the macula; the evolution of this form can be very rapid, causing loss of central vision (visual acuity <1/10) in a few weeks or months.

In one embodiment, the age-related macular degeneration (AMD) is selected from early AMD onset, dry AMD, and wet AMD. Preferably, the age-related macular degeneration (AMD) is wet AMD.

Apart from age, AMD risk factors further comprise heredity (genetic predisposition), the iris coloration and the exposure to light.

In one embodiment, the subject is an elderly subject, that is an aging subject past the stage of maturity. In one embodiment, an elderly human subject is of at least 45, at least 50 or at least 55 years old.

The posology may vary within a wide range depending on the therapeutic and/or nutraceutical indication and the route of administration, and also the general health, age, sex, and body weight of the individual. The skilled artisan will be able to determine appropriate dosages depending on these and other factors.

In one embodiment, the composition or the pharmaceutical composition is orally administered to the subject at least once per week, at least two or three times per week or at least once daily. For instance, the subject may ingest the content of a 10mL or 20mL vial comprising the composition or the pharmaceutical composition as described above at least once a day.

In one embodiment, the herbal composition or the pharmaceutical composition may be administered prior to and/or after at least one eye surgery for preventing and/or treating an ocular degenerative disease.

The herbal composition or the pharmaceutical composition may be administered sequentially to or simultaneously with at least one second pharmaceutical composition that is suitable for preventing and/or treating an ocular degenerative disease, typically AMD.

In one embodiment, the at least one second pharmaceutical composition comprises an anti-VEGF treatment. In one embodiment, the anti-VEGF treatment is selected from the group consisting of ranibizumab, pegaptanib, aflibercept, brolucizumab and a combination thereof. In one embodiment, the anti-VEGF treatment is selected from the group consisting of ranibizumab, pegaptanib, aflibercept, brolucizumab and a combination thereof. In one embodiment, the anti-VEGF treatment is ranibizumab.

In one embodiment, the at least one second pharmaceutical composition comprises verterporfin in the context of a photodynamic treatment.

### EXAMPLES

The present invention is further illustrated by the following examples.

### Example 1: Chicken egg ChorioAllantoic Model (CAM)

Example 1 shows the anti-neoangiogenetic effects of a composition comprising *Alpinia galanga and Phyllantus emblica* extracts in a chicken egg CAM model.

During this experiment, the herbal composition was deposited on the surface of the chorioallantoic membrane in a surface delimited by a ring. The herbal composition was tested after dilutions to 1/5 and 1/10 in PBS medium.

Depositing only PBS was used as negative control.

No significant toxicity of the herbal composition was observed. A strong inhibition and regression of the vascular network of the embryo even at the first day of the treatment.

The herbal composition according to the invention has an anti-angiogenic character, even at a 1/10 dilution.

### Example 2: Preparation of a herbal composition according to the invention

3.5 kg of *Alpinia Galanga* roots from the company ALP ERBO (43 boulevard des tilleuls F04100 Manosque) were crushed, then introduced into a solid /liquid extractor in the presence of 8 liters of 96*%* ethanol (Carbo ERBA CAS number: 64-17-5).

After 1 hour at 60°C, the mixture was filtered and the alcoholic solution was concentrated under reduced pressure, yielding 230 grams of a pasty fragrant extract.

The extraction operation was repeated with the same plant residue wherein 5 liters of ethanol were added. After filtration and concentration, 270 grams of a pasty extract were obtained. The overall extraction yield is 14*%* by mass.

The Triphala extract mixture was commercially available from the company L'HORIZON NATURE (88, rue Paul Bert - 69400 Villefranche sur Saône).

100 mg of the first composition (*A. galanga* extract), and 450 mg of the second composition (Triphala mix) were mixed, yielding a herbal composition.

### Example 3: Pre-clinical toxicity assessment

The subacute toxicity of a herbal composition comprising *Alpinia galanga* and *Phyllanthus emblica* was assessed at 300 mg / kg *in vivo.*

Two groups of mice (8 weeks old, male and female) were processed, 6-8 mice in each group weighing between 22 and 42 g each. The oral administration of the herbal composition was carried out by gavaging following OECD guidelines No. 407.
- Group 1: served as a control group receiving normal saline solution for 28 days,
- Group 2: received the herbal composition daily at a dose of 300 mg/kg per body weight for 28 days.

The monitoring of toxicological parameters indicative of any sub-acute toxicity was carried out on the batches studied during the 4 weeks of the experimental protocol.

Animals are observed individually at least once during the first 30 minutes and regularly during the first 24 hours after treatment. Special care should be taken during the first 4 hours and daily for 28 days after administration of the substance.

No mortality or behavioral alteration was reported for the two products tested during the 28 days of subacute administration.

The macroscopic inspection of the internal organs after the study revealed no alteration. The renal and hepatic histological alterations were reported at the study group.

No significant difference in the biochemical parameters relating to hepatic lesions (ASAT, ALAT, etc.), renal dysfunction (urea, creatinine, etc.) was demonstrated after the administration of the dose of 300 mg/kg.

### Example 2: Preparation of a fermented herbal composition according to the invention

The herbal composition of Example 1 was diluted with 2.5 liters of water. 5 g of glucose and 5 g of ammonium nitrate were added in the obtained suspension. The suspension was vigorously stirred, followed by adding 40 g of brewer's yeast (*Saccharomyces cerevisiae*).

The suspension inoculated with *Saccharomyces cerevisiae* was left under vigorous stirring for 7 days, providing a fermented herbal composition according to the invention.

### Example 3: Clinical observation after administering the herbal composition according to the invention

The test concerns the inclusion of 16 patients, 8 women and 8 men, whose average age was 83.25 years old. One patient left the study on his own and another did not participate to the follow-up.

Data were obtained from 14 patients, 13 of whom presented exudative (wet) AMD and 1 presenting strong myopic neo-vessels (severe short-sightedness). The inclusion criteria included the presence of exudative AMD and/or Visual Acuity greater than or equal to 4/10.

Each patient was seen in consultation for diagnosis or follow-up. Measuring visual acuity and OCT (optical coherence tomography) were performed at each consultation.

Ampoules comprising the herbal composition according to the invention were distributed to the subjects for 1 month with the recommendation of taking one ampoule per day.

Each patient was summoned 15 days after the first dose and after stopping the treatment with the composition of the invention.

The administration was initiated at 3 different stages of the overall ocular treatment as follows.
- 3 subjects received the ampoules before initiating the 1st anti-VEGF intravitreal injection (IVT).
- 7 received the ampoules between 2 series of IVT (one series comprising 3 IVT at a rate of 1 per month).
- 3 received the ampoules during the IVT inclusion phase.
- 1 only received the ampoules without any other treatment (neither sequential nor concomitant).

After a month of treatment as detailed above, the following effects were observed:
- 100*%* disappearance of scotomas.
- 75*%* reduction in metamorphopsia.
- A perceived visual improvement of 57*%*.
- Two to three letters gained in visual acuity.
- No significant anatomical change is visible on OCT exams.
- Among the 3 patients who did not receive another competitive ampoule treatment, 1 described a clear improvement in vision quantified at an additional 1/10 at 1 month from treatment, the other 2 did not feel any improvement. Their visual acuity remained identical.
- 80*%* of patients treated could recommend the product to a loved one.
   2 subjects were reviewed at 3 months and 5 months after stopping the blisters. Their visual acuity was stable.

## Claims

1. A herbal composition comprising:
a) a first composition comprising an extract of *Alpinia galanga,* and
b) a second composition comprising an extract of *Phyllanthus emblica*, and optionally further comprising *Terminalia chebula* and *Terminalia bellirica* extracts.

2. The herbal composition according to claim **1**, wherein the first, and/or the second composition, are fermented compositions with at least one yeast, preferably a yeast of the Saccharomycetaceae family, even more preferably the yeast is *Saccharomyces cerevisiae.*

3. The herbal composition according to claim **1** or claim **2**, said herbal composition being a fermented herbal composition with at least one yeast, preferably a yeast of the Saccharomycetaceae family, even more preferably the yeast is *Saccharomyces cerevisiae.*

4. The herbal composition according to any one of claims **1** to **3**, said composition comprising
- from 1 g/L to 70 g/L of the first composition consisting of an extract of *Alpinia galanga,* and
- from 1 g/L to 70 g/L of the second composition comprising an extract of *Phyllanthus emblica.*

5. The herbal composition according to any one of claims **1** to **4**, said composition comprising
- from 1 g/L to 70 g/L of the first composition consisting of an extract of *Alpinia galanga*, and
- from 10 g/L to 70 g/L of the second composition consisting of a mixture of *Phyllanthus emblica, Terminalia chebula* and *Terminalia bellirica* extracts

6. A pharmaceutical composition comprising the herbal composition according to any one of claims **1** to **5** and at least one pharmaceutically acceptable excipient.

7. A nutraceutical composition comprising the herbal composition according to any one of claims **1** to **5** and at least one nutraceutically acceptable excipient.

8. The pharmaceutical composition according to claim **6**, or the nutraceutical composition according to claim **7**, wherein said pharmaceutical or nutraceutical composition is a liquid composition formulated in the form a vial packaging.

9. The herbal composition according to any one of claims **1** to **5** or the pharmaceutical composition according to claim **6**, for use as a drug.

10. The herbal composition according to any one of claims **1** to **5** or the pharmaceutical composition according to claim **6**, for use in the treatment of an ocular degenerative disease selected from the group consisting of age-related macular degeneration (AMD), severe short-sightedness, cataract, diabetic retinopathy, and glaucoma.

11. The composition for use according to claim **10**, wherein the ocular degenerative disease is age-related macular degeneration (AMD) selected from the group consisting of early AMD, dry AMD and wet AMD, preferably AMD is wet AMD.

12. The composition for use according to any one of claims **9** to **11**, wherein the herbal composition or the pharmaceutical composition is orally administered to the subject at least once a day.

13. The composition for use according to any one of claims **10** to **12**, wherein the herbal composition or the pharmaceutical composition is administered sequentially to or simultaneously with at least one second pharmaceutical composition being selected from i) an anti-VEGF treatment, preferably the anti-VEGF treatment being selected from ranibizumab, pegaptanib, aflibercept and brolucizumab and a combination thereof and/or ii) verterporfin.

14. A process for preparing a herbal composition according to any one of claims **1** to **5**, comprising the steps of:
i. supplying a first composition comprising an extract of *Alpinia galanga,*
ii. supplying a second composition comprising an extract of *Phyllanthus emblica*, and optionally further comprising *Terminalia chebula* and *Terminalia bellirica* extracts,
iii. mixing the first and the second compositions, leading to a herbal composition.

15. The process according to claim **14**, further comprising the steps of:
iv. fermenting the herbal composition of step (iii) with at least one yeast, preferably at least one Saccharomycetaceae strain, even more preferably *Saccharomyces cerevisiae,* leading to a fermented herbal composition,
v. optionally condensing the fermented herbal composition of step (v), leading to a condensed herbal composition, and
vi. optionally mixing the fermented herbal composition of step (iv) or the condensed herbal composition of step (v) with at least one pharmaceutically and/or nutraceutically acceptable excipient.
